# FASCICULE DE BREVET EUROPEEN

(11) **EP 2 376 529 B1**
(45) Date de publication et mention de la délivrance du brevet: **30.03.2016**
(21) Numéro de dépôt: 10703307.8
(22) Date de dépôt: 07.01.2010
(51) Int. Cl.: C07K 14/47, C07K 5/08, A61K 38/43, A61K 8/64

(54) **NOUVEAUX PEPTIDES ANTI-AGE ET COMPOSITION COSMETIQUE ET/OU PHARMACEUTIQUE LES CONTENANT**
NEUE ANTI-AGING-PEPTIDE UND KOSMETISCHE UND/ODER PHARMAZEUTISCHE ZUSAMMENSETZUNG DAMIT
NOVEL ANTI-AGEING PEPTIDES AND COSMETIC AND/OR PHARMACEUTICAL COMPOSITION CONTAINING SAME

(30) Priorité: 09.01.2009 FR 0900066
(43) Date de publication de la demande: 19.10.2011
(73) Titulaire: ISP Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR); IMBERT, Isabelle, F-75010 Paris (FR)
(74) Mandataire: Macquet, Christophe
(86) Numéro de dépôt international: PCT/FR2010/000006
(87) Numéro de publication internationale: WO 2010/079284

(56) Documents cités:
- EP-A- 1 864 997
- WO-A-99/57137
- HIRAYAMA JUN ET AL: "Structural and functional features of transcription factors controlling the circadian clock" CURRENT OPINION IN GENETICS & DEVELOPMENT, vol. 15, no. 5, octobre 2005 (2005-10), pages 548-556, XP002545453 ISSN: 0959-437X
- KONDRATOV ROMAN V: "A role of the circadian system and circadian proteins in aging" AGEING RESEARCH REVIEWS, vol. 6, no. 1, mai 2007 (2007-05), pages 12-27, XP002545454 ISSN: 1568-1637
- TANIOKA MIKI ET AL: "Molecular Clocks in Mouse Skin" JOURNAL OF INVESTIGATIVE DERMATOLOGY, [Online] vol. 129, no. 5, 27 novembre 2008 (2008-11-27), pages 1225-1231, XP002545455 ISSN: 0022-202X

### SEQUENCE LISTING

<110> ISP Investments Inc
<120> NOUVEAUX PEPTIDES ANTI-AGE ET COMPOSITION COSMETIQUE ET/OU PHARMACEUTIQUE LES CONTENANT
<130> Bv PCT 09-114
<150> FR 0900066
   <151> 2009-01-09
<160> 6
<170> PatentIn version 3.3
<210> 1
   <211> 7
   <212> PRT
   <213> Artificial
<220>
   <223> Synthetic peptide
<220>
   <221> MOD_RES
   <222> (7)..(7)
   <223> AMIDATION
<400> 1
   Tyr Val Ser Thr Pro Tyr Asn
   1 5
<210> 2
   <211> 5
   <212> PRT
   <213> Artificial
<220>
   <223> Synthetic peptide
<220>
   <221> MOD_RES
   <222> (5)..(5)
   <223> AMIDATION
<400> 2
   Val Ser Thr Pro Glu
   1 5
<210> 3
   <211> 3
   <212> PRT
   <213> Artificial
<220>
   <223> Synthetic peptide
<220>
   <221> MOD_RES
   <222> (3)..(3)
   <223> AMIDATION
<400> 3
   Ser Thr Pro
   1
<210> 4
   <211> 5
   <212> PRT
   <213> Artificial
<220>
   <223> Synthetic peptide
<220>
   <221> MOD_RES
   <222> (5)..(5)
   <223> AMIDATION
<400> 4
   Leu His Ser Thr Pro
   1               5
<210> 5
   <211> 6
   <212> PRT
   <213> Artificial
<220>
   <223> Synthetic peptide
<220>
   <221> MOD_RES
   <222> (6)..(6)
   <223> AMIDATION
<400> 5
   Arg His Ser Thr Pro Glu
   1               5
<210> 6
   <211> 5
   <212> PRT
   <213> Artificial
<220>
<223> Synthetic peptide
<220>
   <221> MOD_RES
   <222> (5)..(5)
   <223> AMIDATION
<400> 6
   His Ser Thr Pro Glu
   1               5

## Description

La présente invention se situe dans les domaines de la cosmétique et de la dermopharmacie. La présente invention se rapporte à des composés peptidiques de séquences spécifiques de formule générale (I) R₁-(AA)ₙ- X₁ - Ser - Thr - Pro - X₂-(AA)ₚ - R₂ destinés à restaurer le rythme circadien et à resynchroniser l'horloge biologique des cellules de la peau, ainsi que pour prévenir et corriger les effets du vieillissement.

La fonction première de l'épiderme, est de constituer une barrière entre l'environnement extérieur et le milieu intérieur. C'est la couche la plus externe de l'épiderme, *le stratum corneum,* qui assure cette fonction. Il est composé de kératinocytes au stade ultime de leur différenciation, les cornéocytes, scellés les uns aux autre par un épais ciment intercellulaire, à la fois souple et imperméable. Cette barrière physique qu'est la peau permet au corps humain de se protéger de nombreux types d'agressions. Ces agressions peuvent avoir diverses origines intrinsèques, telles que le vieillissement chronologique ou encore des modifications biochimiques qui ont lieu lors d'états de fatigue, de stress, de changements hormonaux comme lors de la grossesse.... D'autres agressions elles, ont une origine extrinsèque, telle que la pollution, le soleil, les maladies.....En réponse à ces agressions, l'aspect de la peau est modifié et l'on voit apparaître alors des rides et ridules, des taches d'hyper ou d'hypo-pigmentation, une sécheresse voire une déshydratation de la peau, un amincissement de l'épiderme, une élastose, des imperfections, des taches de vieillesse.... Ces modifications sont dues à l'altération des fonctions de renouvellement cellulaire, de cohésion cellulaire, de synthèse de collagène, d'élastine et d'autres protéines et conduisent finalement à une diminution des qualités de barrière protectrice de la peau et à un aspect peu esthétique de celle-ci.

La peau comme tous les autres organes, est soumise à l'influence de variations périodiques. Elle est le siège d'une organisation cohérente de rythmes circadiens et nycthéméraux modulant divers cycles biologiques, le plus souvent de grande amplitude (F. Henry et al., Rev Med Liege; 57 : 10 : 661-665). Ces rythmes cutanés chez l'homme suggèrent que le jour, la peau privilégie diverses fonctions protectrices vis-à-vis de l'environnement. En soirée et la nuit, elle favorise son renouvellement cellulaire et divers processus métaboliques de synthèse.

Un sujet en bonne santé, dont l'organisme vit en harmonie avec son environnement, présente une synchronisation de ses rythmes biologiques. En revanche, des perturbations des rythmes biologiques peuvent apparaître dans un certain nombre de conditions dites de désynchronisation (Reinberg et Touitou, 1996). Une désynchronisation est un état où deux variables rythmiques (ou plus), antérieurement synchronisées, ont cessé de présenter les mêmes relations de fréquence et/ou d'acrophase et montrent des relations temporelles différentes des relations habituelles. La désynchronisation peut être d'origine externe, elle dépend alors des modifications de l'environnement et se retrouve, par exemple, lors d'un vol trans- méridien de cinq fuseaux horaires (phénomène du jet-lag) ou dans le travail de nuit. La désynchronisation d'origine interne, quant à elle, ne dépend pas des facteurs de l'environnement. On la retrouve dans le vieillissement ou dans un certain nombre de maladies telles que la dépression ou certains cancers. Ces troubles de la désynchronisation peuvent être corrigés par divers traitements : par exemple l'administration de lumière forte pour traiter la dépression saisonnière, ou encore l'administration de mélatonine (Dijk et coll., 1995 ; Eastman et Miescke, 1990 ; Palm et coll., 1991 ; Shochat et coll., 1998 ; Touitou et coll., 1998). En cosmétique, il est bien connu que l'efficacité d'un soin peut être optimisée selon le moment de la journée durant lequel il est administré. Par exemple, les crèmes de jour permettent de protéger la peau des agressions extérieures durant la journée, alors que les crèmes de nuit permettent à la peau de réparer les dommages subis pendant la journée par augmentation du renouvellement et du métabolisme cellulaires. En outre, il a été démontré que le vieillissement s'accompagne d'une modification des rythmes biologiques, avec une diminution de l'amplitude et une tendance à l'avance de phase (Weinert D., Chronobiol. Int. 2000 ; 17 :261-83). De plus, il a été montré que l'horloge circadienne était altérée par ce même vieillissement. Cependant, aucun traitement ne permet, à l'heure actuelle, à la peau de « réinitialiser » ou encore de relancer son cycle circadien, ni de resynchroniser son horloge biologique. Un tel traitement permettrait ainsi d'aider la peau à se remettre d'un décalage horaire, d'un travail de nuit, ou encore de lutter contre les signes dus au vieillissement.

De manière surprenante, la Demanderesse a découvert que des composés peptidiques de formule générale suivante R₁-(AA)ₙ- X₁ -Ser - Thr - Pro - X₂ - (AA)ₚ- R₂, avaient la propriété de restaurer le rythme circadien et de resynchroniser l'horloge biologique des cellules de la peau. Aucun document de l'art antérieur ne décrit de tels composés peptidiques permettant d'obtenir de tels effets. Par ailleurs, ces composés peptidiques sont caractérisés par le fait qu'ils sont des agents activateurs de la protéine Clock (Circadian Locomotor Output Cycles Kaput) et ce de manière directe ou indirecte, qui est impliquée dans la régulation du cycle circadien. Par conséquent, la présente invention a pour objet des composés peptidiques activateurs de Clock ainsi que leur utilisation dans des compositions cosmétiques et pharmaceutiques afin de restaurer le rythme circadien et de resynchroniser l'horloge biologique des cellules de la peau ou encore de prévenir ou corriger les signes dus au vieillissement.

On sait depuis longtemps que l'horloge circadienne est contrôlée par une boucle négative de régulation impliquant un ensemble de gènes, notamment les gènes Per-1 (Period), Clock et BMAL-1 (Brain and Muscle ARNt-like Protein). Jusqu'à présent, dans l'art antérieur, de nombreuses applications ont déjà été proposées quant à l'utilisation des nucléotides et/ou de protéines issues des gènes Clock, Per-1 ou BMAL-1. On peut citer par exemple le brevet US 6 291 429 qui décrit l'utilisation du produit du gène Clock pour la resynchronisation du cycle du sommeil ou de processus physiologiques ou endocriniens, la resynchronisation du corps après un décalage horaire.... Les documents brevets WO 99/57237 et EP1864997 révèlent des protéines issues des gènes Clock, Per-1 et BMAL-1 pour la régulation des rythmes biologiques. Cependant, aucun document de l'art antérieur ne décrit l'utilisation de peptides spécifiques activateurs de Clock pour restaurer l'horloge biologique interne de la peau.

La présente invention a pour premier objet des composés peptidiques de formule (1) suivante

R₁-(AA)ₙ- X₁ - Ser - Thr - Pro - X₂ - (AA)ₚ- R₂

dans laquelle,
X₁ représente une thréonine, une sérine, ou est égal à zéro,
X₂ représente une isoleucine, une leucine, une valine, une alanine, une glycine, ou est égal à zéro,
AA représente un acide aminé quelconque à l'exception de la proline, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 13 résidus d'acides aminés,
caractérisé en ce qu'il correspond à une des formules suivantes:

(SEQ ID n°1) Tyr - Val - Ser - Thr - Pro - Tyr- Asn- NH₂

(SEQ ID n°2) Val - Ser - Thr - Pro -Glu - NH₂

(SEQ ID n°3) Ser - Thr - Pro - NH₂

(SEQ ID n°4) Leu -His - Ser - Thr - Pro - NH₂

(SEQ ID n°5) Arg -His - Ser - Thr - Pro -Glu - NH₂

(SEQ ID n°6) His - Ser - Thr - Pro -Glu - NH₂,

et permet de restaurer le rythme circadien et resynchroniser l'horloge biologique des cellules de la peau.

La présente invention a pour second objet une composition cosmétique comprenant des composés peptidiques de formule générale (I) de séquence SEQ ID n°1-6.

Le premier objet de l'invention se rapporte à un composé peptidique de formule générale (I) suivante:
dans laquelle,

R₁-(AA)ₙ- X₁ - Ser - Thr - Pro - X₂ - (AA)ₚ-R₂

dans laquelle,
X₁ représente une thréonine, une sérine, ou est égal à zéro,
X₂ représente une isoleucine, une leucine, une valine, une alanine, une glycine, ou est égal à zéro,
AA représente un acide aminé quelconque à l'exception de la proline, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 13 résidus d'acides aminés,
caractérisé en ce qu'il correspond à une des formules suivantes:

(SEQ ID n°1) Tyr - Val - Ser - Thr - Pro - Tyr- Asn- NH₂

(SEQ ID n°2) Val - Ser - Thr - Pro -Glu - NH₂

(SEQ ID n°3) Ser - Thr - Pro - NH₂

(SEQ ID n°4) Leu -His - Ser - Thr - Pro - NH₂

(SEQ ID n°5) Arg -His - Ser - Thr - Pro -Glu - NH₂

(SEQ ID n°6) His - Ser - Thr - Pro -Glu - NH₂,

et permet de restaurer le rythme circadien et resynchroniser l'horloge biologique des cellules de la peau.

Le composé peptidique selon l'invention est caractérisé en ce qu'il est activateur des produits des gènes du rythme circadien. Par « gènes du rythme circadien » on entend les gènes Clock, Per-1 et BMAL-1.

Dans un mode de réalisation particulier, la séquence du composé peptidique est la séquence SEQ ID n°3, c'est-à-dire Ser - Thr - Pro - NH₂.

Dans un autre mode de réalisation, la séquence du composé peptidique est la séquence SEQ ID n°4, c'est-à-dire Leu -His - Ser - Thr - Pro - NH₂.

Le terme « peptide » ou « composé peptidique » désigne un enchaînement de deux ou plusieurs acides aminés liés entre eux par des liaisons peptidiques ou par des liaisons peptidiques modifiées.

Par « peptide » ou « composé peptidique », il faut entendre le peptide naturel ou synthétique de l'invention tel que décrit ci-dessus, qu'il soit obtenu par protéolyse ou de manière synthétique.

Le peptide de formule générale (I) selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide selon l'invention peut être d'origine naturelle ou synthétique. Préférentiellement selon l'invention, le peptide est obtenu par synthèse chimique.

Enfin, le principe actif peut être un peptide unique de séquence SEQ ID n°1-6, ou un mélange de ces peptides.

Le composé peptidique selon l'invention peut être utilisé à titre de médicament.

Le second objet de la présente invention se rapporte à des compositions cosmétiques comprenant ledit composé peptidique de formule générale (I) de séquence SEQ ID n°1-6. De manière préférée, les compositions selon l'invention se présentent sous une forme adaptée à l'application par voie topique comprenant un milieu cosmétiquement ou dermatologiquement acceptable. Par « cosmétiquement ou dermatologiquement acceptable », on entend des milieux qui conviennent à une utilisation en contact avec la peau ou les phanères humains, sans risque de toxicité, d'incompatibilité, d'instabilité, de réponse allergique et autres. Préférentiellement, ledit composé peptidique est présent dans la composition à une concentration comprise entre 0,0005 et 500 ppm environ, et préférentiellement à une concentration comprise entre 0,01 et 5 ppm. Dans les compositions selon l'invention, le composé peptidique est préalablement solubilisé dans un ou plusieurs solvants cosmétiquement ou dermatologiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux, le principe actif selon l'invention est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur physiologiquement acceptable.

Les compositions destinées à être appliquées sur la peau peuvent se présenter sous forme de solution aqueuse ou hydro- alcoolique, d'émulsion eau dans huile ou huile dans eau, de microémulsion, de gel aqueux ou anhydre, de sérum, ou encore de dispersion de vésicules, de patch, de crème, de spray, d'onguent, de pommade, de lotions, de colloïde, de solution, de suspension ou autres. Les compositions peuvent aussi être appliquées sur les phanères sous forme de shampoing, de teinture ou de mascara à appliquer au pinceau ou au peigne, en particulier sur les cils, les sourcils ou les cheveux, ou encore de soins pour les ongles tels que les vernis.

Dans un mode de réalisation particulier, la composition selon l'invention contient en outre, au moins un autre principe actif favorisant l'action dudit principe actif peptidique. On peut citer, de manière non limitative, les classes d'ingrédients suivantes : d'autres agents actifs peptidiques, des extraits de végétaux, des agents cicatrisants, anti-âge, anti-rides, apaisants, anti-radicalaire, anti-UV, des agents stimulant la synthèse de macromolécules dermiques ou le métabolisme énergétique, des agents hydratants, anti-bactériens, anti-fongiques, anti-inflammatoires, anesthésiques, des agents modulants la différenciation, la pigmentation ou la dépigmentation cutanée, des agents stimulants la pousse des ongles ou des cheveux.... Préférentiellement, on utilisera un agent anti-radicalaire ou antioxydant, ou un agent stimulant la synthèse de macromolécules dermiques, ou encore un agent stimulant le métabolisme énergétique.

En outre, des additifs tels que des agents épaississants, émulsifiants, humectants, émollients, des parfums, des antioxydants, des agents filmogènes, chélatants, séquestrants, conditionnants....peuvent être ajoutés à la composition.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Un troisième objet de la présente invention se rapporte au composé peptidique de séquence SEQ ID n°1-6 en tant que principe actif activateur de Clock. On entend par peptide « activateur de Clock», tout peptide selon l'inventioncapable d'augmenter l'activité de Clock, soit par augmentation de la synthèse protéique de Clock (par modulation directe ou indirecte de l'expression génique de Clock) soit par d'autres processus biologiques tels que la stabilisation de la protéine Clock ou encore la stabilisation des transcrits d'ARN messager.

Un autre objet de la présente invention se rapporte à une composition comprenant ledit composé peptidique de formule générale (I) de séquence SEQ ID n°1-6 selon ladite invention pour restaurer le rythme circadien et resynchroniser l'horloge biologique des cellules de la peau.

Une réalisation particulière de l'invention concerne une telle composition pour activer le renouvellement cellulaire et stimuler le métabolisme cellulaire. Plus particulièrement, l'invention consiste en une telle composition pour réduire les dysfonctionnements causés par le décalage horaire et/ou le travail de nuit. Avantageusement, l'invention concerne ledit composé peptidique de séquence SEQ ID n°1-6 utilisé dans une composition destinée à respecter la chronobiologie cutanée notamment par une application en soin cosmétique la nuit.

Une autre réalisation de l'invention consiste en une composition comprenant ledit composé peptidique de formule générale (I) de séquence SEQ ID n°1-6 pour prévenir ou traiter les signes cutanés du vieillissement. Les « signes cutanés du vieillissement » incluent, mais ne se limitent pas à, toutes les manifestations visibles sur la peau causés par le vieillissement. On entend notamment, les rides, ridules, crevasses, pores élargis, imperfections, perte de fermeté, décoloration, tâches de vieillesse, kératoses, perte de collagène, et autres changements du derme et de l'épiderme....Par « signes cutanés du vieillissement », on entend également toutes modifications de l'aspect extérieur de la peau et des phanères dues au vieillissement comme, par exemple, les rugosités superficielles de la couche cornée, les rides et ridules, mais également toute modification interne de la peau qui ne se traduit pas systématiquement par un aspect extérieur modifié comme, par exemple, l'amincissement du derme ou toute autre dégradation interne de la peau consécutive à une exposition aux rayonnements ultraviolets (UV). Plus particulièrement, l'invention se rapporte à la composition telle que décrite précédemment utilisée afin de réduire les signes de fatigue de la peau.

On applique topiquement sur la peau ou les phanères à traiter une composition contenant une quantité efficace de principe actif peptidique pour restaurer le rythme circadien et resynchroniser l'horloge biologique des cellules.

Par ailleurs, la composition est appliquée avant le coucher de façon à respecter le rythme circadien de la peau pour obtenir un effet anti-vieillissant sur la peau. En effet, durant la nuit, la peau privilégie des fonctions de renouvellement ainsi que des processus métaboliques de synthèse. Par conséquent, l'application de la composition telle que revendiquée, en respectant le rythme biologique de la peau permet d'obtenir un effet anti-vieillissant, stimulant le renouvellement cellulaire, régénérant et réduisant ainsi les signes du vieillissement.

Les exemples suivants décrivent et démontrent l'efficacité de composés peptidiques tels que décrits selon l'invention. Les formulations cosmétiques citées sont représentatives de l'invention mais sont données uniquement à titre d'illustration et ne doivent pas être interprétés comme des limitations de la présente invention.

### Exemple 1: Mise en évidence de l'effet activateur du peptide SEQ ID n°2 sur l'expression de la protéine Clock sur des fibroblastes en culture.

Une étude de l'effet activateur du peptide SEQ ID n°2, a été réalisée en évaluant l'expression des protéines Clock en western blot sur des fibroblastes en culture. La technique de western blotting est une méthode semi-quantitative qui permet d'apprécier le taux de protéines Clock dans les cellules.

### Protocole

Des fibroblastes en cultures sont cultivés dans des boîtes de diamètre 100 mm à 37°C dans une atmosphère humidifiée contenant 5% de CO2 pendant 18 h en présence ou non du peptide SEQ ID n°2, dilué 1% (à partir d'une solution 10⁻⁴M). Les cellules sont rincées puis détachées du support à l'aide d'un tampon d'extraction (20mM TRIS, 150mM NaCl, 10mM EDTA, 0.2% Triton X10) en présence d'un cocktail d'inhibiteurs de protéases (Sigma). Les protéines ainsi extraites, sont centrifugées à 4°C à 10000 rpm pendant 10 minutes avant d'être dosées par le kit de dosages des protéines BCA (Pierce). Les .lysats cellulaires sont mélangés à un tampon de dénaturation et soumis à une électrophorèse SDS-PAGE. Le gel utilisé est un Nupage 4-12% (invitrogen). Les protéines sont enfin transférées sur une membrane de nitrocellulose (Pal corporation). Les membranes sont saturées dans du PBS-lait 5%, 0,1% tween 20, 2 h à température ambiante, puis incubées à 4°C toute la nuit avec un anticorps primaire anti-Clock au 1/1000^{ème} (ABcam) suivie d'une incubation avec un anticorps secondaire anti-lapin Iggy-peroxydase dilué au 1/5000^{ème}. La révélation est effectuée par un substrat chimioluminescent. L'évaluation quantitative des protéines présentes dans les cellules est effectuée grâce au logiciel chemiimager (Alpha innotech Corporation USA). La quantité des protéines est exprimée en pourcentage d'intensité lumineuse comparée à la condition contrôle qui n'a pas eu le traitement.

### Résultats :

Les résultats obtenus montrent que le traitement par le peptide SEQ ID n°2, dilué à 1% augmente de façon significative la protéine Clock dans les fibroblastes en culture. Les résultats sont résumés dans le tableau ci-dessous.

| Intensité (%) | Expérience 1 |
|---|---|
| Contrôle non traités | 100% |
| 1% traitement | 154% |

L'expérience a été réalisée plusieurs fois et démontre par le test statistique du t Student que l'augmentation de l'expression de la protéine Clock est significative (p = 0,0445).

### Conclusion:

Le peptide SEQ ID n°2 permet d'augmenter l'expression de la protéine Clock sur des cellules dermiques en culture.

### Exemple 2: Mise en évidence de l'effet activateur du peptide SEQ ID n°3 sur l'expression de la protéine Clock, Per-1 et BMAL-1 sur des biopsies de peau.

Une étude de l'effet activateur du peptide SEQ ID n°3, a été réalisée en évaluant l'expression de la protéine Clock, Per-1 et BMAL-1 sur des biopsies de peaux *ex vivo.*

### Protocole

Des études ex vivo par immunomarquage ont permis de mettre en évidence l'expression de la protéine Clock, Per-1, et BMAL-1 sur des échantillons de peau. Des échantillons de peau humaine, sont mis en culture à l'interface air/liquide. Le peptide SEQ ID n°3 dilué à 1% (à partir d'une solution 10⁻⁴M).est appliqué de façon topique sur ces échantillons pendant 48 h.

Des échantillons de peau non traitées par le peptide SEQ ID n°3 servent de contrôle. Les échantillons de peaux sont fixés au formaldéhyde 10% et inclus dans la paraffine. Des coupes de 3 µm sont réalisées au microtome. L'immunomarquage est effectué après un déparaffinage et un démasquage des sites antigéniques. L'immunomarquage s'effectue pour la protéine Clock, par un anticorps (ABcam) dilué au 1/250ème et un anticorps secondaire au 1/50^{ème} couplé à un fluorochrome.

L'immunomarquage s'effectue pour la protéine Per-1, par un anticorps polyclonal (Cosmobio) dilué au 1/100^{ème} et un anticorps secondaire au 1/50^{ème} couplé à un fluorochrome.

L'immunomarquage s'effectue pour la protéine BMAL-1, par un anticorps polyclonal (Aviva Systems Biology) dilué au 1/120^{ème} et un anticorps secondaire au 1/50^{ème} couplé à un fluorochrome.

Les lames sont alors montées dans un milieu adéquat et observées au microscope à épifluorescence (Nikon Eclipse E600).

### Résultats :

Les résultats obtenus montrent que les peaux traitées avec le peptide SEQ ID n°3 expriment des quantités de protéine Clock, Per-1 et BMAL-1 supérieures aux peaux non traitées.

L'évaluation semi-quantitative du marquage est effectuée par observation microscopique et résumée dans le tableau ci-dessous.

| | Non traité | Traité par peptide SEQ ID n°3 dilué à 1% |
|---|---|---|
| Marquage protéine Clock | +/- | + |
| Marquage protéine PER-1 | + | +++ |
| Marquage BMAL-1 | + | +++ |

### Conclusion :

L'administration de peptide SEQ ID n°3 permet d'augmenter l'expression des protéines Clock, Per-1 et BMAL-1sur une peau irradiée et traitée.

### Exemple 3 : Mise en évidence de l'effet activateur du peptide SEQ ID n°3 sur l'expression de la protéine Clock, sur des biopsies de peau au cours du vieillissement

Une étude de l'effet activateur du peptide SEQ ID n°3, a été réalisée en évaluant l'expression de la protéine Clock sur des modèles de peaux, vieillis artificiellement en culture. L'analyse s'est effectuée après 62 h et 134 h de culture.

### Protocole

Des études ex vivo par immunomarquage ont permis de mettre en évidence l'expression de la protéine Clock, sur des échantillons de peau vieillis en culture. Des échantillons de peau humaine, sont mis en culture à l'interface air/liquide, pendant 62 h et 134 h. Le peptide SEQ ID n°1 dilué à 1% (à partir d'une solution 10⁻⁴M).est appliqué de façon topique sur ces échantillons à raison de 2 fois par jour pendant le temps de l'expérience.

Des échantillons de peau non traitées par le peptide SEQ ID n°3 servent de contrôle. Les échantillons de peaux sont fixés au formaldéhyde 10% et inclus dans la paraffine. Des coupes de 3 µm sont réalisées au microtome. L'immunomarquage est effectué après un déparaffinage et un démasquage des sites antigéniques. L'immunomarquage s'effectue pour la protéine Clock, par un anticorps anti-rabbit (ABcam) dilué au 1/250ème et un anticorps secondaire au 1/50^{ème} couplé à un fluorochrome.

Les lames sont alors montées dans un milieu adéquat et observées au microscope à épifluorescence (Nikon Eclipse E600).

### Résultats :

Les résultats obtenus montrent que le marquage de la protéine Clock (essentiellement épidermique) est augmenté après 62 heures de culture, par rapport aux lames témoins non traitées. Cette augmentation perdure dans le temps puisqu'au bout de 134 heures, le marquage de la protéine Clock est fortement augmenté par rapport aux peaux témoins non traitées. Le tableau ci-dessous donne une évaluation visuelle du marquage.

| | Non traité | Traité par peptide SEQ ID n°3 dilué à 1% |
|---|---|---|
| Marquage protéine Clock 62 h | + | ++ |
| Marquage protéine Clock 134 h | - | ++ |

### Conclusion :

L'application du composé peptidique SEQ ID n°3 permet d'augmenter l'expression de la protéine Clock sur des biopsies de peau traitées et vieillies artificiellement en culture.

### Exemple 4 : Mise en évidence de l'effet anti-vieillissement du peptide SEQ ID n°3 sur des fibroblastes en culture.

Une étude de l'effet anti-vieillissement du peptide SEQ ID n°3, a été réalisée en évaluant l'activité de la béta-galactosidase sur des fibroblastes en culture à court et long terme. En effet, la béta-galactosidase est connue pour être présente dans les cellules sénescentes alors qu'on ne trouve pas d'activité béta-galactosidase dans les cellules pré-sénescentes, quiescentes ou immortelles.

### Protocole

Des fibroblastes en cultures dans des labteck 8 puits sont maintenus 2 semaines (effet court terme) ou 7 semaines (effet long terme) en présence ou non du peptide SEQ ID n°3, dilué 1% (à partir d'une solution 10⁻⁴M). Le traitement est effectué une fois par jour. Les cellules non traitées mais maintenues en culture pendant 2 ou 7 semaines selon les besoins de l'expérience servent de contrôle. Le jour du marquage, les cellules sont rincées, fixées dans un mélange (2% glutaraldéhyde - 2% formaldéhyde) pendant 3 minutes. Les cellules sont rincées et on applique 300 µl de (5-bromo-4-chloro-3 indolyl b-D-galactosidase) appelé communément X-gal (substrat de la béta-galactosidase). L'incubation s'effectue 24 h dans l'incubateur à CO2, puis les cellules sont rincées et la labteck est rapidement montée dans un milieu adéquat. L'observation s'effectue au microscope à transmission. Le principe est simple, lorsque les cellules sont sénescentes et contiennent la béta-galactosidase, le substrat X-gal est clivé en un produit bleu insoluble.

### Résultats :

L'activité béta-galactosidase est mesurée dans les cellules témoins non traitées par une coloration de celles-ci en bleues. L'activité béta-galactosidase est fortement réduite dans les cellules traitées par le peptide SEQ ID n°3. Cet effet est observé à la fois dans les cellules traitées à court terme (2 semaines d'expérience) mais également à long terme lorsque les cellules sont traitées pendant 7 semaines consécutives.

### Conclusion :

L'administration du peptide SEQ ID n°3 permet de mettre en évidence un effet anti-vieillissement sur des fibroblastes en culture vieillis artificiellement pendant 2 ou 7 semaines.

### Exemple 5 : Préparation d'une crème anti-âge

| ***Noms commerciaux*** | ***Noms INCI*** | % ***massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n° 2 | | 1 ppm |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

### Mode Opératoire:

Préparer et fondre la phase A à 65-70°C. Chauffer la phase C à 65-70°C. La phase B est ajoutée à la phase Ajuste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

## Revendications

1. Composé peptidique de formule générale suivante (I) :
R₁-(AA)ₙ- X₁ - Ser - Thr - Pro - X₂ - (AA)ₚ - R₂
dans laquelle,
X₁ représente une thréonine, une sérine, ou est égal à zéro,
X₂ représente une isoleucine, une leucine, une valine, une alanine, une glycine, ou est égal à zéro,
AA représente un acide aminé quelconque à l'exception de la proline, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, substituée par un groupement protecteur pouvant être choisi parmi une chaîne alkyle de C₁ à C₂₀, ou un groupement NH2, NHY ou NYY avec Y représentant une chaîne alkyle de C₁ à C₄, ladite séquence de formule générale (I) étant constituée de 3 à 13 résidus d'acides aminés, **caractérisé en ce qu'**il correspond à une des formules suivantes :
(SEQ ID n°1) Tyr - Val - Ser - Thr - Pro - Tyr - Asn - NH₂
(SEQ ID n°2) Val - Ser - Thr - Pro - Glu - NH₂
(SEQ ID n°3) Ser - Thr - Pro - NH₂
(SEQ ID n°4) Leu - His - Ser - Thr - Pro - NH₂
(SEQ ID n°5) Arg - His - Ser - Thr - Pro - Glu - NH₂
(SEQ ID n°6) His - Ser - Thr - Pro - Glu - NH₂,
et permet de restaurer le rythme circadien et resynchroniser l'horloge biologique des cellules de la peau.

2. Composé peptidique selon la revendication 1, **caractérisé en ce qu'**il est utilisé à titre de médicament.

3. Composition cosmétique comprenant à titre de principe actif ledit composé peptidique selon la revendication 1.

4. Composition cosmétique selon la revendication 3, **caractérisée en ce qu'**elle se présente sous une forme adaptée à l'application par voie topique comprenant un milieu cosmétiquement acceptable.

5. Composition cosmétique selon l'une des revendications 3 ou 4, **caractérisée en ce que** ledit composé peptidique est présent dans la composition à une concentration comprise entre 0,0005 et 500 ppm environ, et préférentiellement à une concentration comprise entre 0,01 et 5 ppm.

6. Composition cosmétique selon l'une quelconque des revendications 3 à 5, **caractérisée en ce que** ledit principe actif peptidique est solubilisé dans un ou plusieurs solvants, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

7. Composition cosmétique selon l'une quelconque des revendications 3 à 6, **caractérisée en ce qu'**elle contient en outre, au moins un autre principe actif favorisant l'action dudit principe actif peptidique choisi parmi un agent anti-radicalaire ou antioxydant, ou un agent stimulant la synthèse de macromolécules dermiques, ou encore un agent stimulant le métabolisme énergétique.

8. Composé peptidique tel que défini dans la revendication 1, en tant que principe actif activateur de Clock.

9. Composition selon l'une quelconque des revendications 3 à 7 pour restaurer le rythme circadien et resynchroniser l'horloge biologique des cellules de la peau.

10. Composition selon la revendication 9, pour activer le renouvellement cellulaire et stimuler le métabolisme cellulaire.

11. Composition selon la revendication 9, pour réduire les dysfonctionnements causés par le décalage horaire et/ou le travail de nuit.

12. Composition selon l'une quelconque des revendications 3 à 7, pour prévenir ou traiter les signes cutanés du vieillissement, et réduire les signes de fatigue de la peau.

13. Composition selon l'une quelconque des revendications 9 à 12, **caractérisé en ce qu'**elle est appliquée sur la peau ou les phanères à traiter avant le coucher de façon à respecter le rythme circadien de la peau.

## Patentansprüche

1. Peptidverbindung mit der folgenden allgemeinen Formel (I) :
R₁- (AA) ₙ- X₁ - Ser - Thr - Pro - X₂ - (AA)ₚ - R₂
wobei
X₁ ein Threonin, ein Serin darstellt oder gleich null ist, X₂ ein Isocleucin, ein Leucin, ein Valin, ein Alanin, ein Glycin darstellt oder gleich null ist.
AA irgendeine Aminosäure mit Ausnahme von Prolin oder einem seiner Derivate darstellt und
n und p ganze Zahlen zwischen 0 und 4 sind,
R₁ die primäre Aminofunktion der N-terminalen Aminosäure darstellt, frei oder substituiert durch eine Schutzgruppe, die aus einer Acetylgruppe, einer Benzoylgruppe, einer Tosylgruppe oder einer Benzyloxycarbonylgruppe gewählt werden kann,
R₂ die Hydroxylgruppe der Carboxylfunktion der C-terminalen Aminosäure darstellt, frei oder substituiert durch eine Schutzgruppe, die aus einer Alkylkette mit C₁ bis C₂₀ oder einer NH2-, NHY- oder NYY-Gruppe gewählt werden kann, wobei Y eine Alkylkette mit C₁ bis C₄ darstellt, wobei die Sequenz mit der allgemeinen Formel (I) aus 3 bis 13 Rückständen von Aminosäuren besteht, **dadurch gekennzeichnet, dass** sie einer der folgenden Sequenzen entspricht:
(SEQ ID Nr. 1) Tyr - Val - Ser - Thr - Pro - Tyr - Asn -NH₂
(SEQ ID Nr. 2) Val - Ser - Thr - Pro - Glu - NH₂
(SEQ ID Nr. 3) Ser - Thr - Pro - NH₂
(SEQ ID Nr. 4) Leu - His - Ser - Thr - Pro - NH₂
(SEQ ID Nr. 5) Arg - His - Ser - Thr - Pro - Glu - NH₂
(SEQ ID Nr. 6) His - Ser - Thr - Pro - Glu - NH₂,
und ermöglicht, die circadiane Rhythmik wiederherzustellen und die biologische Uhr der Zellen der Haut neu zu synchronisieren.

2. Peptidverbindung nach Anspruch 1, **dadurch gekennzeichnet, dass** sie als Medikament verwendet wird.

3. Kosmetische Zusammensetzung, umfassend als Wirkstoff die Peptidverbindung nach Anspruch 1.

4. Kosmetische Zusammensetzung nach Anspruch 3, **dadurch gekennzeichnet, dass** sie eine Form aufweist, die für die Anwendung auf topischem Weg ausgelegt ist, umfassend ein annehmbares kosmetisches Medium.

5. Kosmetische Zusammensetzung nach einem der Ansprüche 3 oder 4, **dadurch gekennzeichnet, dass** die Peptidverbindung in der Zusammensetzung in einer Konzentration die ungefähr zwischen 0,0005 und 500 ppm liegt, und vorzugsweise in einer Konzentration zwischen 0,01 und 5 ppm vorhanden ist.

6. Kosmetische Zusammensetzung nach einem der Ansprüche 3 bis 5, **dadurch gekennzeichnet, dass** der Peptidwirkstoff in einem oder in mehreren Lösemitteln gelöst ist, wie z.B. Wasser, Glycerol, Ethanol, Propylenglycol, Butylenglycol, Dipropylenglycol, ethoxylierten oder propoxylierten Diglykolen, cyclischen Polyolen, Vaselin, einem pflanzlichen Öl oder jeder Mischung dieser Lösemittel.

7. Kosmetische Zusammensetzung nach einem der Ansprüche 3 bis 6, **dadurch gekennzeichnet, dass** sie außerdem mindestens einen weiteren Wirkstoff umfasst, der die Wirkung des Peptidwirkstoffs begünstigt, ausgewählt aus einem Radikalfänger oder Antioxidationsmittel oder einem Mittel, das die Synthese von Hautmakromolekülen stimuliert oder auch einem Mittel, das den Energiestoffwechsel stimuliert.

8. Peptidverbindung, wie in Anspruch 1 definiert, als Clock-Aktivator-Wirkstoff.

9. Zusammensetzung nach einem der Ansprüche 3 bis 7, um die circadiane Rhythmik wiederherzustellen und die biologische Uhr der Zellen der Haut neu zu synchronisieren.

10. Zusammensetzung nach Anspruch 9, um die Zelleneuerung zu aktivieren und den Zellstoffwechsel zu stimulieren.

11. Zusammensetzung nach Anspruch 9, um die Funktionsstörungen zu reduzieren, die von der zeitlichen Versetzung und/oder der Nachtarbeit hervorgerufen werden.

12. Zusammensetzung nach einem der Ansprüche 3 bis 7, um die Anzeichen der Hautalterung zu verhindern oder zu behandeln und die Anzeichen der Hautermüdung zu reduzieren.

13. Zusammensetzung nach einem der Ansprüche 9 bis 12, **dadurch gekennzeichnet, dass** sie auf die Haut oder die Hautanhangsgebilde, die behandelt werden sollen, vor dem Schlafen aufgebracht wird, um die circadiane Rhythmik der Haut zu respektieren.

## Claims

1. Peptide compound having the following general formula (I):
R₁- (AA) ₙ-X₁-Ser-Thr-Pro-X₂- (AA) ₚ-R₂
wherein,
X₁ is a threonine, a serine, or is equal to zero,
X₂ is an isoleucine, a leucine, a valine, an alanine, a glycine, or is equal to zero,
AA is any amino acid with the exception of proline, or a derivative thereof, and n and p are integers between 0 and 4,
R₁ is the primary amine function of the N-terminal amino acid, either free or substituted by a protective group which can be selected from an acetyl group, a benzoyl group, a tosyl group or a benzyloxycarbonyl group,
R₂ is the hydroxyl group of the carboxyl function of the C-terminal amino acid, substituted by a protective group which can be selected from a C₁ to C₂₀ alkyl chain or an NH2, NHY or NYY group where Y is a C₁ to C₄ alkyl chain, said sequence having general formula (I) consisting of 3 to 13 amino acid residues,
**characterised in that** it corresponds to one of the following formulae:
(SEQ ID no 1)Tyr-Val-Ser-Thr-Pro-Tyr-Asn-NH₂
(SEQ ID no 2)Val-Ser-Thr-Pro-Glu-NH₂
(SEQ ID no 3)Ser-Thr-Pro-NH₂
(SEQ ID no 4)Leu-His-Ser-Thr-Pro-NH₂
(SEQ ID no 5)Arg-His-Ser-Thr-Pro-Glu-NH₂
(SEQ ID no 6)His-Ser-Thr-Pro-Glu-NH₂
and suitable for restoring the circadian rhythm and resynchronising the biological clock of skin cells.

2. Peptide compound according to claim 1, **characterised in that** it is used as a medicine.

3. Cosmetic composition comprising, as an active ingredient, said peptide compound according to claim 1.

4. Cosmetic composition according to claim 3, **characterised in that** it is presented in a form suitable for topical application comprising a cosmetically or dermatologically acceptable medium.

5. Cosmetic composition according to any of claims 3 or 4, **characterised in that** said peptide compound is present in the composition at a concentration between approximately 0.0005 and 500 ppm, and preferably at a concentration between 0.01 and 5 ppm.

6. Cosmetic composition according to any of claims 3 to 5, **characterised in that** said peptide active ingredient is solubilised in one or a plurality of solvents, such as water, glycerol, ethanol, propylene glycol, butylene glycol, dipropylene glycol, ethoxylated or propoxylated diglycols, cyclic polyols, petroleum jelly, a vegetable oil or any mixture of these solvents.

7. Cosmetic composition according to any of claims 3 to 6, **characterised in that** it further contains at least one active ingredient promoting the action of said peptide active ingredient chosen from an anti-radical or antioxidant agent or an agent stimulating the synthesis of dermal macromolecules, or else an agent stimulating the energy metabolism.

8. Peptide compound as defined in claim 1, as a clock activation active ingredient.

9. Composition according to any of claims 3 to 7 for restoring the circadian rhythm and resynchronising the biological clock of skin cells.

10. Composition according to claim 9, for activating and stimulating the cellular metabolism.

11. Composition according to claim 9, for reducing the dysfunctions caused by jet lag and/or nightshift work.

12. Composition according to any of claims 3 to 7, for preventing or treating the skin signs of ageing, and reducing the signs of skin fatigue.

13. Composition according to any of clams 9 to 12, **characterised in that** it is applied to the skin or skin appendages to be treated before going to sleep so as to respect the circadian rhythm of the skin.
